# EUROPEAN PATENT SPECIFICATION

(11) **EP 2 598 475 B1**
(45) Date of publication and mention of the grant of the patent: **02.04.2014**
(21) Application number: 12706691.8
(22) Date of filing: 13.02.2012
(51) Int. Cl.: C07C 213/08, C07C 227/16

(54) **PROCESS FOR THE SYNTHESIS OF DICLOFENAC CHOLINE SALT**
VERFAHREN ZUR SYNTHESE VON DICLOFENAC-CHOLINSALZEN
PROCÉDÉ DE SYNTHÈSE DE SEL DE CHOLINE DE DICLOFÉNAC

(30) Priority: 13.07.2011 IT MI20111302
(43) Date of publication of application: 05.06.2013
(73) Proprietor: FARMAKA S.r.l., I-20123 Milano (IT)
(72) Inventor: DI SCHIENA, Michele Giuseppe, I-20087 Robecco sul Naviglio MI (IT)
(74) Representative: Montelatici, Linda Anna
(86) International application number: PCT/IB2012/050641
(87) International publication number: WO 2013/008104

(56) References cited:
- EP-A2- 0 521 393
- FINI A. ET AL.: "Factors governing the dissolution of diclofenac salts", EUROPEAN JOURNAL OF PHARMACEUTICAL SCIENCES, vol. 4, 1996, pages 231-238, XP002661350,

## Description

The present invention relates to a process for the synthesis of diclofenac choline salt, i.e. (2-hydroxyethyl)trimethylammonium [o-(2,6-dichloroanilino)phenyl]acetate, of formula (I).

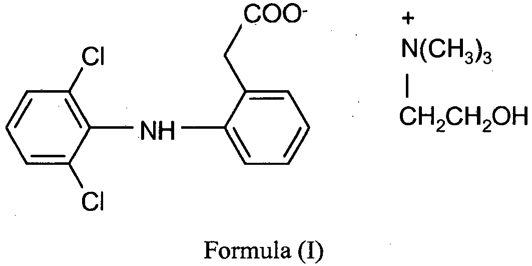

Patent EP0521393 in the name of the Applicant discloses diclofenac choline salt, in the following indicated with the name diclofenac choline, and a process for the production thereof. The process described in said patent comprises the reaction of diclofenac and choline hydroxide, in water or in a suitable organic solvent such as methanol. The reaction in water of said reagents in conditions of a molar excess of diclofenac is also described, and the following purification of the diclofenac choline product by recrystallization from acetone.

A first drawback of said known process consists in that a recrystallization of the obtained product is necessary, since it contains large amounts of impurities deriving from the reagent choline hydroxide.

A second drawback of said known process consists in that said reagent requires particular precautions during transport, storing and manipulation, for example the use of seal containers, gloves and masks for protection of skin, eyes and of the respiratory system, suitably ventilated rooms and extraction of the ammonia vapors deriving from the reagent.

The need for the afore mentioned recrystallization and precautions has a negative effect on the total production time and cost of diclofenac choline.

Besides, even if maintained in a sealed container, choline hydroxide undergoes degradation and carbonation processes which reduce its salting ability in short time, thus further reducing the purity of the obtained final product.

Further, due to the presence of ammonia impurities in the reagent, the process described in the above mentioned patent shall be preferably carried out with a diclofenac excess, which causes an increase of the costs of production of the salt.

It is therefore an object of the present invention to provide a process, which is free from said disadvantages. Said object is achieved with a process, whose main features are disclosed in the first claim, and a solution whose main features are specified in claim 9. Further features of the process according to the present invention are specified in the remaining claims.

The process according to the present invention advantageously provides the desired product, diclofenac choline, having a high purity and in a quantitative yield. The very high purity of the product obtained by the process according to the present invention results in the possibility to avoid further purification steps, which are necessary in the process according to the prior art.

Besides, the process according to the present invention allows obtaining a solution of the desired product in ethanol which, being free from impurities, can be used directly for preparing pharmaceutical compositions. For example, said ethanol solution can be directly used for the preparation of soft capsules or hard capsules for oral administration, since ethanol does not influence the stability of the material of which the capsules are made and helps the pharmaceutical product absorption.

Diclofenac choline obtained with the process according to the invention can be isolated in solid form, for example by evaporation of the ethanol that can be easily recovered and reused in a subsequent production cycle, with a notable economical advantage.

The process for producing (2-hydroxyethyl)trimethylammonium [o-(2,6-dichloroanilino)phenyl]acetate according to the present invention comprises a step of reaction between o-(2,6-dichloroanilino)phenyl]acetic acid, (2-hydroxyethyl)trimethylammonium chloride and a metal hydroxide selected in the group consisting of the alkaline and alkaline-earth metals, in a reaction solvent. Preferably, said reaction is carried out by using equimolar amounts of o-(2,6-dichloroanilino)phenyl]acetic acid, (2-hydroxyethyl)trimethylammonium chloride and metal hydroxide. In this way, it is not necessary to purify the product from excesses of unreacted starting materials.

As reaction solvent, ethanol is used, since by using said reaction solvent, the reaction by-products may be eliminated simply by filtering the reaction mixture. As a matter of fact, in the process according to a preferred embodiment of the invention, said reaction between o-(2,6-dichloroanilino)phenyl]acetic acid, (2-hydroxyethyl)trimethylammonium chloride and metal hydroxide is followed by a filtering step in order to remove reaction products that are not soluble in the reaction solvents, such as for example sodium chloride.

Said filtration advantageously provides a very pure solution that contains (2-hydroxyethyl)trimethylammonium [o-(2,6-dichloroanilino)phenyl]acetate in ethanol, which may be directly used for the preparation of pharmaceutical compositions, such as for filling soft or hard capsules for oral administration.

Besides, it has been found that ethanol as solvent in the process according to the present invention allows to obtain the final product with excellent yields and to isolate (2-hydroxyethyl)trimethylammonium [o-(2,6-dichloroanilino)phenyl]acetate of high purity. Preferably, ethanol having a purity of 95 volume % or higher is used.

Reagents of pharmaceutical grade are advantageously used in the process according to the present invention. The (2-hydroxyethyl)trimethylammonium chloride that is used generally has a purity of 98% of higher on anhydrous basis.

Also said [o-(2,6-dichloroanilino)phenyl]acetic acid used in the process according to the present invention has a purity of 99% or higher on anhydrous basis.

Sodium or potassium hydroxide is preferably used as metal hydroxide in the process according to the present invention.

The process according to the present invention is carried out at a temperature of between 10°C and 40°C. Preferably, said reaction step is carried out at a temperature of between 15°C and 25°C.

Further advantages and features of the according to the present invention will become clear to those skilled in the art from the description of the following non-limiting examples.

### Example 1

4 g (0.1 mol) of sodium hydroxide, 13.96 g (0.1 mol) of (2-hydroxyethyl)trimethylammonium chloride and 29.61 g (0.1 mol) of [o-(2,6-dichloroanilino)phenyl]acetic acid were dissolved in 170 ml of ethanol 95%, by operating at a temperature between 18°C and 20°C under stirring.

The mixture was then filtered and the separated crystalline solid was portionwise washed with ethanol 95%, so as to obtain a total volume of filtered solution of 200 ml.

The solution was kept under stirring for about 3 hours so as to make sure that the salification reaction was complete.

The clear solution was directly used as described in example 3 and 4.

### Example 2

The solution obtained in example 1 was dried by removing the ethanol solvent through evaporation at reduced pressure. 38 g of (2-hydroxyethyl)trimethylammonium [o-(2,6-dichloroanilino)phenyl]acetate were obtained a white crystalline solid. M.p. 178.8-179.7 (DSC). NMR Spectroscopy confirmed the above mentioned structure.

### Example 3

The solution obtained in example 1 was dispensed in hard capsules provided by Ely lilly. Each capsule contained 50 mg of (2-hydroxyethyl)trimethylammonium [o-(2,6-dichloroanilino)phenyl]acetate and about 0,34 ml of ethanol.

### Example 4

The solution obtained in example 1 was dispensed in soft capsules provided by Gelfipharma International (Lodi-Italy). Each capsule contained 50 mg of (2-hydroxyethyl)trimethylammonium [o-(2,6-dichloroanilino)phenyl]acetate, 0,34 ml of ethanol and 10 mg of soja lecithin.

## Claims

1. A process for the production of (2- hydroxyethyl)trimethylammonium [o-(2,6-dichloroanilino)phenyl] acetate, **characterized by** comprising a step of reaction of [o-(2,6-dichloroanilino)phenyl]acetic acid, (2-hydroxyethyl)trimethylammonium chloride and a metal hydroxide selected in the group consisting of alkaline and alkaline-earth metal hydroxides, wherein ethanol is used as reaction solvent.

2. A process according to claim 1, **characterized in that** equimolar amounts of [o-(2,6-dichloroanilino)phenyl]acetic acid, (2-hydroxyethyl)trimethylammonium chloride and metal hydroxide are used in said step of reaction.

3. A process according to claim 1, **characterized in that** said (2-hydroxyethyl)trimethylammonium chloride has a purity of at least 98% on anhydrous basis.

4. A process according to claim 1, **characterized in that** said ethanol has a purity of at least 95%.

5. A process according to claim 1, **characterized in that** said [o-(2,6-dichloroanilino)phenyl]acetic acid has a purity of at least 99% on anhydrous basis.

6. A process according to claim 1, **characterized in that** said metal hydroxide is sodium or potassium hydroxide.

7. A process according to claim 1, **characterized in that** said step of reaction is carried out at a temperature between 10°C and 40°C, preferably between 15°C and 25°C.

8. A process according to claim 1 **characterized in that** said step of reaction is followed by a step of filtration in order to remove reaction products that are insoluble in the solvent.

## Patentansprüche

1. Ein Verfahren zur Herstellung von [o-(2-Hydroxyethyl)-Trimethylammonium-[o-(2,6-Dichloranilin)-Phenyl}-Essigsäure, **gekennzeichnet durch** den Schritt der Reaktion von [o-(2,6-Dichloranilin)-Phenyl]-Essigsäure, (2-Hydroxyethyl)-Trimethylammoniumchlorid und einem Metallhydroxid, ausgewählt aus der Gruppe bestehend aus Alkali-und Erdalkalimetallhydroxiden, wobei Ethanol als ein Reaktions-Lösungsmittel verwendet wird.

2. Ein Verfahren nach Anspruch 1, **dadurch gekennzeichnet, dass** in dem besagten Reaktionsschritt äquimolare Mengen von [o-(2,6-Dichloranilin)-Phenyl]-Essigsäure, (2-Hydroxyethyl)-Trimethylammoniumchlorid und Metallhydroxid verwendet werden.

3. Ein Verfahren nach Anspruch 1, **dadurch gekennzeichnet, dass** das besagte (2-Hydroxyethyl)-Trimethylammoniumchlorid eine Reinheit von wenigstens 98 % hat, auf einer wasserfreien Basis.

4. Ein Verfahren nach Anspruch 1, **dadurch gekennzeichnet, dass** das besagte Ethanol eine Reinheit von wenigstens 95 % hat.

5. Ein Verfahren nach Anspruch 1, **dadurch gekennzeichnet, dass** die besagte [o-(2,6-Dichloranilin)-Phenyl]-Essigsäure eine Reinheit von wenigstens 99 % hat, auf einer wasserfreien Basis.

6. Ein Verfahren nach Anspruch 1, **dadurch gekennzeichnet, dass** das besagte Metallhydroxid Natrium- oder Kaliumhydroxid ist.

7. Ein Verfahren nach Anspruch 1, **dadurch gekennzeichnet, dass** der besagte Reaktionsschritt bei einer Temperatur zwischen 10 °C und 40 °C durchgeführt wird, vorzugsweise zwischen 15 °C und 25 °C.

8. Ein Verfahren nach Anspruch 1, **dadurch gekennzeichnet, dass** auf den besagten Reaktionsschritt ein Filtrationsschritt folgt, um Reaktionsprodukte zu entfernen, die in dem Lösungsmittel unlöslich sind.

## Revendications

1. Procédé de production de [o-(2,6-dichloro-anilino)phényl]acétate de (2-hydroxyéthyl)triméthylammonium, **caractérisé en ce qu'**il comprend une étape de réaction d'acide [o-(2,6-dichloroanilino)phényl]acétique, de chlorure de (2-hydroxyéthyl)triméthylammonium et d'hydroxyde de métal choisi dans le groupe constitué par les hydroxydes de métal alcalin et de métal alcalino-terreux, dans lequel de l'éthanol est utilisé en tant que solvant réactionnel.

2. Procédé selon la revendication 1, **caractérisé en ce que** des quantités équimolaires d'acide [o-(2,6-dichloro-anilino)phényl]acétique, de chlorure de (2-hydroxyéthyl)-triméthylammonium et d'hydroxyde de métal sont utilisées dans ladite étape de réaction.

3. Procédé selon la revendication 1, **caractérisé en ce que** ledit chlorure de (2-hydroxyéthyl)triméthylammonium a une pureté d'au moins 98 %, sur une base anhydre.

4. Procédé selon la revendication 1, **caractérisé en ce que** ledit éthanol a une pureté d'au moins 95 %.

5. Procédé selon la revendication 1, **caractérisé en ce que** ledit acide [o-(2,6-dichloroanilino)phényl]acétique a une pureté d'au moins 99 %, sur une base anhydre.

6. Procédé selon la revendication 1, **caractérisé en ce que** ledit hydroxyde de métal est l'hydroxyde de sodium ou de potassium.

7. Procédé selon la revendication 1, **caractérisé en ce que** ladite étape de réaction est réalisée à une température comprise entre 10 °C et 40 °C, de préférence entre 15 °C et 25 °C.

8. Procédé selon la revendication 1, **caractérisé en ce que** ladite étape de réaction est suivie par une étape de filtration afin d'éliminer les produits réactionnels qui ne sont pas solubles dans le solvant.
